## Europäisches Patentamt

## European Patent Office

## Office européen des brevets

(19)

(11) Publication number: **0 122 748**
**B1**

(12)

# EUROPEAN PATENT SPECIFICATION

(45) Date of publication of patent specification: **23.12.87**

(51) Int. Cl.⁴: **A 61 M 1/34**

(21) Application number: **84302278.1**

(22) Date of filing: **03.04.84**

(54) **Blood filter.**

(30) Priority: **08.04.83 US 483375**

(43) Date of publication of application:
**24.10.84 Bulletin 84/43**

(45) Publication of the grant of the patent:
**23.12.87 Bulletin 87/52**

(84) Designated Contracting States:
**DE FR GB IT NL SE**

(56) References cited:
**GB-A-2 022 434**
**US-A-3 765 536**
**US-A-3 993 461**
**US-A-4 115 277**
**US-A-4 140 635**

(73) Proprietor: **SHILEY INCORPORATED**
**17600 Gillette**
**Irvine California (US)**

(72) Inventor: **Servas, Francis Martin**
**31882 Paseo Labranza**
**San Juan Capistrano California (US)**
Inventor: **Gremel, Robert Franklin**
**5621 Selkirk Drive**
**Huntington Beach California (US)**
Inventor: **Ryan, Timothy Charles**
**25221 York Circle**
**Laguna Hills California (US)**

(74) Representative: **Graham, Philip Colin Christison et al**
**Pfizer Limited Ramsgate Road**
**Sandwich, Kent CT13 9NJ (GB)**

Courier Press, Leamington Spa, England.

## Description

This invention is concerned with a filter unit for the purification of blood and especially with a cardiotomy reservoir containing the filter unit.

Blood supplied to a patient must usually be purified by filtration to avoid jeopardizing the patient. The blood may be obtained from the patient during surgery when it is advantageous to store excess blood outside the body to facilitate the surgical procedure, or in blood conservation by scavenging the blood from the wound. Such blood is usually collected in a cardiotomy reservoir and purified thereby passage through a filter unit within the reservoir. In cardiopulmonary bypass procedures where an extracorporeal blood circuit is generated and an oxygenator in the circuit takes over the function of the lungs, a cardiotomy reservoir commonly supplies purified blood to the oxygenator. The blood passing through the reservoir must not only be purified of undesirable particulate matter such as surgical debris, but must also be freed of entrained air bubbles before being returned to the patient or supplied to an oxygenator.

It is known to provide in a cardiotomy reservoir a filter unit comprising means for screening out particulate matter, and means for defoaming the blood to remove the air trapped therein. Examples of such known devices include those disclosed in U.S. Patents No. 3,507,395 and 3,768,653. U.S. Patent 3,507,395 to Bentley discloses a cardiotomy reservoir comprising a chamber containing a plate surrounded by a fibrous filter element contained in a nylon bag. The plate first spreads the incoming blood to remove air bubbles therefrom which are vented, or drawn by vacuum, from the chamber, and the filter removes solid particles from the blood as it passes therethrough before leaving the chamber. U.S. Patent 3,768,653 to Brumfield discloses a cardiotomy reservoir comprising a tubular chamber having a tangential inlet for the blood which is directed onto a filter across one end of the chamber which also contains a conical air filter.

Many other filtration systems are known for filtering blood and many use multiple layer elements to remove unwanted materials from the blood as it passes through the layers. U.S. Patents 3,765,536 and 3,765,537 to Rosenberg disclose multiple layer blood filter elements including one comprising a first layer of coarse polypropylene netting, a second downstream layer of open-mesh polyester, a third spacer layer of polypropylene netting, a fourth microporous layer and a fifth polypropylene netting spacer layer.

GB—A—2022434 discloses a cardiotomy reservoir containing a perforated cylinder extending from one end of the reservoir to the other. The cylinder houses a filter and first defoaming means for removing large bubbles and carries externally a second defoaming means for removing small bubbles.

US—A—3993461 describes a similar reservoir in which blood passes through the perforations of a hollow member and defoamer filter.

Other commercially available reservoirs contain various arrangements of defoamer and filter layers. However, the known arrangements suffer from the disadvantage of having a limited useful life before one or more of the layers, particularly the filter layers, becomes blocked or obstructed. We have now discovered an arrangement whereby the useful lifetime of the cardiotomy reservoir may be extended beyond that hitherto possible without significant blockage or obstruction in normal use and which more efficiently separates entrained gas and particulate matter from the blood.

According to the invention there is provided a combined cardiotomy defoamer, filter and reservoir device for removing air bubbles and undesirable particulate matter from blood evacuated from a surgical field, said device accommodating substantial blood flow for extended periods of time with minimal pressure increase in said device between the blood inlet and outlet of said device, comprising a hollow, tubular defoamer/filter element, a generally hollow housing surrounding said defoamer/filter element, and having a blood inlet in communication with the interior of said defoamer/filter element, a reservoir chamber for receiving the filtered blood after it has been filtered through said defoamer/filter element, an air vent for venting air separated from the blood in said device, and a blood outlet in communication with said reservoir chamber, said defoamer/filter element comprising a first annular tube of reticulated porous foam material treated with antifoam compound for removing gas bubbles from said blood and an annular tube of a fine pore size filter screen medium for removing undesirable particulate matter surrounding said first annular tube characterized in that said defoamer/filter element further comprises a second annular tube of a nonwoven fibrous depth filter medium surrounding and closely proximate said first annular tube for providing a first stage filter for larger particulate matter, and a third annular tube of porous material surrounding said second annular tube and positioned between and closely proximate said second annular tube and said annular tube of a fine pore size filter screen medium for providing a porous spacing between the external surface of said second annular tube and said downstream fourth annular tube of a fine pore size filter screen medium to provide multiple fluid paths between the exterior surface of said first stage filter and said second stage filter.

This invention also provides a filter for a cardiotomy reservoir comprising a final stage screen filter having a substantially uniform pore size, preferably selected from the range of from about 20 to 60 microns.

In the invention, the depth filter material is preferably treated with a wetting agent, such as a polyol. This provides unexpectedly rapid flow and wetting in the depth filter and contributes to the efficiency of the device.

By spacing apart the depth filter and screen filter layers and interposed between them at least one spacer layer, filtering efficiency and the useful lifetime of the filter device of the invention is improved. In this arrangement, obstruction of the screen filter by filtered particles and air bubbles in an adjacent depth filter layer is avoided and obstruction of an escape route for air bubbles leaving the depth filter by a blood-soaked adjacent screen filter is also avoided, resulting in a free-flowing system that does not exhibit substantial pressure buildup with time, which would be caused by increasing blockages in the filter.

An embodiment of this invention is illustrated in the accompanying drawings, in which:

Figure 1 is a top view of a cardiotomy reservoir according to the invention;

Figure 2 is a side elevational view of the reservoir shown in Figure 1;

Figure 3 is a partial cross-sectional view of the reservoir shown in Figure 1, taken along the line 3—3;

Figure 4 is a cross-sectional view of the reservoir shown in Figure 2, taken along the line 4—4.

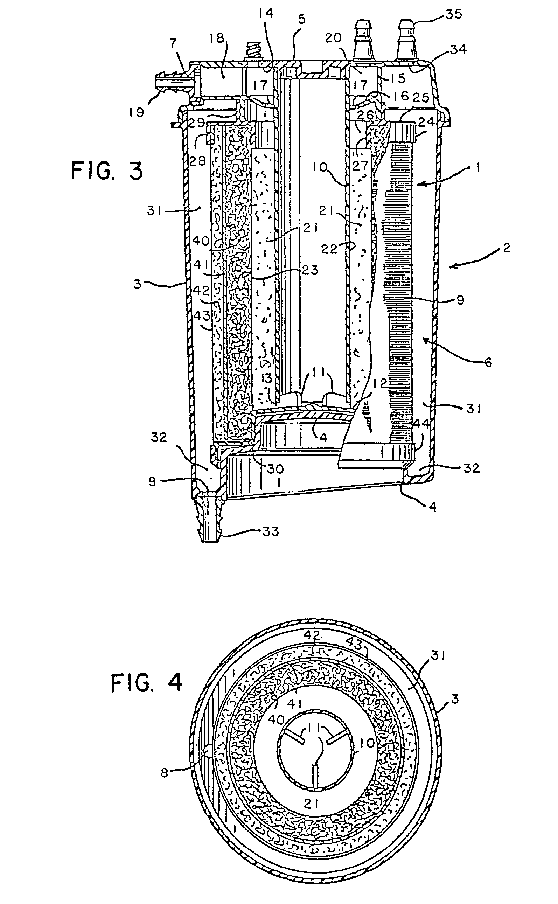

Referring to Figure 3, there is shown a filter unit 1 according to the invention incorporated in a cardiotomy reservoir 2. Reservoir 2 comprises an outer, substantially cylindrical wall 3 having a base 4 and a top 5 to form within them a reservoir chamber 6. The chamber 6 has an inlet port 7 near its top and an outlet 8 at its bottom. A hollow, tubular defoamer/filter element 9 is arranged along the longitudinal axis of the chamber 6 and is spaced from outer wall 3 in a manner such that all blood entering the reservoir 2 through inlet 7 is caused to flow from the hollow space within the defoamer/filter element 9 through its walls before leaving the chamber through outlet 8.

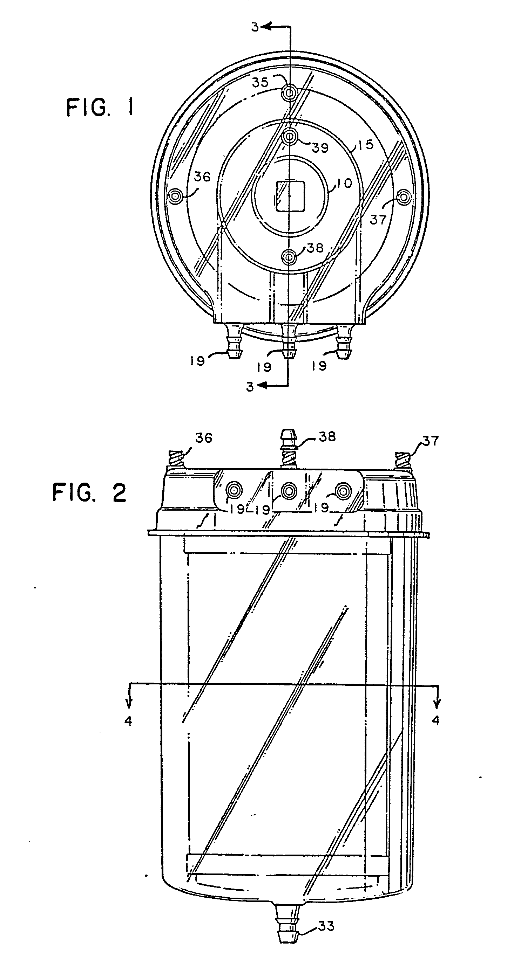

More particularly, element 9 is arranged around an internal, substantially cylindrical column 10 extending along the central, longitudinal axis of chamber 6 between base 4 and top 5. In the embodiment shown in Figure 3, the bottom of column 10 is seated upon spacers 11 which project upwardly from a filter base 12 shaped to nest-against, and be sealed to, the base 4 of reservoir 2. As can be seen more clearly in Figure 4, spacers 11 are arranged radially and symmetrically around the longitudinal axis of the chamber and, as shown in Figure 3, separate the bottom of column 10 from the upper surface of filter base 12 thereby creating a space 13 between the bottom end of column 10 and filter base 12. The top of column 10, however, is attached to the inner surface 14 of top 5 without any significant gap between the top of column 10 and surface 14. An annular wall 15 extends downwardly from the inner surface 14 of top 5 and is spaced around and concentric with the upper end of column 10. Partway down wall 15, an inwardly projecting flange 16 extends part-way towards column 10 so as to leave a gap 17 around column 10. An inlet passage 18 opens through wall 15 above flange 16 and from inlet port 7. Inlet 7 has an external connector 19 for attachment of a tube (not shown) to conduct blood into the reservoir for purification. Usually, as shown in Figures 1 and 2, three identical inlet ports 7 are provided with their respective connectors 19. Each inlet 7 has inlet passages 18 to within wall 15. Column 10, wall 15 and the inner surface 14 of top 5 therefore define an annular inlet chamber 20 around column 10 in fluid communication with inlet port 7 and, through gap 17, as to an extended, annular inlet passage 21 around column 10 and between the outer surface 22 of column 10 and the inner surface 23 of defoamer/filter element 9.

Element 9 is sealably retained at its upper end in an upper end cap 24 which comprises an end plate 25 having a central orifice 26 around column 10 and bounded by a downwardly projecting, inner annular flange 27. The periphery of plate 25 has a downwardly projecting outer, annular flange 28. The separation of flanges 27 and 28 is such as to snugly accept the thickness of defoamer/filter element 9 which is sealed between those flanges and to the underside of plate 25. On the upper surface of plate 25, between orifice 26 and the periphery of plate 25, an upwardly extending, annular flange 29 surrounds and is sealed to the outer surface of the lower part of wall 15, as shown in Figure 3. The lower end of defoamer/filter element 9 is sealably retained in an internal, downward annular step 30 in filter base 12.

Thus, blood entering reservoir 2 through inlet 7 and passing into extended inlet passage 21 must travel through defoamer/filter 9 to reach outlet 8 from the reservoir. The outer surface of defoamer/ filter 9 is separated from outer wall 3 of the reservoir by a space 31 around defoamer/filter 9. Purified blood leaving defoamer/filter 9 passes down space 31 and is guided to outlet 8 by the base 4 of reservoir 2 forming a trough 32 around and below the base of defoamer/filter 9 as a lower extension of space 31. Trough 32 is angled towards outlet 8 to direct the flow of blood through the outlet. Outlet 8 has a connector 33 for attachment of an outlet tube (not shown). Space 31 around defoamer/filter 9 is a fluid communication with a gas vent 34 through the top 5 of reservoir 2 by means of a chamber between outer wall 3 and annular wall 15. Vent 34 has a connector 35 for attachment of an outlet tube (not shown).

The top 5 of reservoir 2 has further fluid access means. A pair of priming ports 36 and 37 (Figure 1) provide means for adding fluid to the reservoir without filtration by accessing gap 31 directly. Another pair of priming ports 38 and 39 (Figures 1 and 2) provide means for adding fluid to the reservoir with filtration by accessing the annular chamber 20 within wall 15 so that the added fluid will pass into extended inlet passage 21 and through defoamer/filter 9.

The defoamer/filter element 9 comprises · a series of concentric tubular members, as can be most clearly seen in Figure 4. Element 9 comprises a first, inner tubular defoamer member 40 whose inner surface 23 defines with central column 10 the vertical confines of the extended annular inlet passage 21. Around defoamer 40 is a second tubular member 41 serving as a depth filter which is surrounded by a third tubular member 42 serving as a spacer which in turn is surrounded by a fourth tubular member 43 serving as a screen filter. The adjacent surfaces of members 40, 41, 42 and 43 are in close proximity and are preferably in contact with one another to form a substantially continuous, layered structure.

Defoamer member 40 comprises an open cell, blood compatible, synthetic polymeric foam material to collapse blood foam as the blood begins to pass through defoamer/filter element 9. Defoamer 40 is preferably formed of a thermally reticulated polyurethane foam having, for example, about a 20 pore per inch size. Preferably, defoamer 40 is treated with a medical antifoam agent to assist the defoaming step. Suitable antifoam agents include silicone antifoams such as Antifoam A available from Dow Chemical Company. Defoamer 40 is preferably formed from a sheet of foam about 6—1/2 inches high and about 12 inches wide to fit a typical reservoir having a capacity of about 2 liters. The two opposite upright shorter sides are joined together to form the tubular member and the longitudinal seam formed by the joint is sealed by radio frequency welding.

Depth filter 41 comprises a synthetic nonwoven material such as polypropylene or a polyester and provides a system of complex pathways as a first stage filter for particulate matter in the blood. While defoamer layer 40 will act as a gross filter to prevent passage of large debris carried by the blood, such as bone chips and tissue fragments, filter 41 more effectively filters and prevents passage of smaller particles in the blood through element 9. Preferably, filter 41 has a mean pore diameter of about 50 microns and a maximum pore diameter of about 90 microns. Filter 41 is also prefereably made up from about 80% 3 denier fiber and about 20% 1.8 denier fiber and callendared to produce a fabric having a weight of about 8 ounces/sq. yard. Preferably, the outer side of filter 41 has a relatively smooth callendared surface and the inner side has a fluffy surface to increase the effective area of the inner surface of the filter which is first exposed to the blood and to better filter gradually the particulate matter without becoming clogged. Filter 41 should wet well and therefore is not treated with antifoam agent but is preferably treated with a small amount of wetting agent. In a typical wetting treatment procedure, the fabric is out to an appropriate sheet size, usually about 6—1/2 inches high and about 13 inches wide, dry cleaned and heat sealed along its edge. The fabric is washed with a cleaning agent in pyrogen-free water and rinsed. After washing and while still damp, the fabric is treated with a solution of polyol wetting agent in pyrogen-free water and thoroughly dried. The final fabric should preferably exhibit thorough wetting when hydrostatically tested at 4.0+1.0 inches of water. The two opposite shorter sides of the fabric are joined together to form the tubular member and the seam is sealed by impulse welding.

Spacer 42 comprises a synthetic foam material similar or identical to that of defoamer 40 but having a larger pore size, usually about double the pore size of defoamer 40, for example about 10 pore per inch foam. Spacer 42 is preferably prepared in the same manner as that described for defoamer 40. Preferably spacer (42) is formed of reticulated porous foam material untreated with any antifoam material. In normal manufacture, the first three layers of element 9 consisting of defoamer 40, depth filter 41 and spacer 42 are assembled in their proper order, one around the other and sealed by hot melt glue at their bottom edges to filter base 12. Outer screen filter 43 is pulled over the assembly and sealed to filter base 12 between base 12 and a retaining ring 44 (Figure 3). The top of the completed defoamer/filter element 9 is then sealed to upper end cap 24 between flanges 27 and 28 and plate 25.

Spacer 42 separates depth filter 41 from the outer layer of the defoamer/filter element 9: the screen filter 43. Filter 43 comprises a thin layer of blood compatible, synthetic material having a small, defined pore size. Preferably, filter 43 is formed from a woven polyester fabric. On the scale described above for the other components of the defoamer/filter element 9, filter 43 is preferably formed from a piece of fabric about 13—1/2 inches wide and about 6—1/2 inches high. The two opposite shorter sides are sewn together and the filter assembled with the remainder of the element 9 as described above. For optimum filtering, filter 43 should have a pore size preferably no less than about 20 microns, for example about 43 microns, and preferably less than 60 microns. Preferably, defoamer/filter assembly 9 is at least about 90% effective in removing from the blood material having a size greater than or equal to about 20 microns.

In use, blood is supplied to reservoir 2 either by a pump (not shown) in the inlet line or by a vacuum pump (not shown) applying suction to reservoir 2. The blood enters the reservoir 2 through inlet port 7 and inlet passage 18 to inlet chamber 20. Then the blood spills over flange 16 through gap 17 and into the extended inlet passage 21. Preferably, flange 16 is angled downwardly, as shown in Figure 3, to direct the blood against the outer surface 22 of column 10. This encourages the blood to cascade down column 10 and tends to minimize splashing and excess foam formation while providing an extended surface area of blood to encourage release of larger air bubbles entrained in the blood. The blood then travels across gap 21 between column 10 and defoamer 40 and enters defoamer 40 where the blood foam is collapsed and substantially all the air bubbles remaining in the blood are separated out before the blood passes into depth filter 41 for removal of particulate matter therefrom. The following spacer layer 42 prevents the blood and any air bubbles leaving filter 41 from obstructing each other and provides separate pathways for gas and blood through the open structure of spacer layer 42. If the spacer 42 was omitted any air bubble leaving depth filter 41 would tend to obstruct the adjacent portion of screen filter 43 and hinder or prevent passage of blood through that portion of screen 43. Similarly, blood leaving depth filter 41 would tend to form a film in screen filter 43 and form a barrier to any air bubble about to leave filter 41, thereby obstructing that portion of filter 41 against passage of blood. Moreover, particulate matter trapped by filter 41 near its outer, downstream surface would also tend to block the adjacent portion of screen filter 43 to passage of blood through the screen.

This spacer 42 provides improved flow of fluid through the defoamer/filter element 9 and reduces blockage in comparison to prior art devices, thereby extending the useful life of the reservoir and avoiding pressure buildup caused by blockages and which can damage the blood.

When blood flows down the outside of screen filter 43, spacer 42 prevents air in the lower part of the filter device from becoming trapped there by that blood. Gas is free to escape up the spacer layer to exit through the filter device near its top into space 31 around the filter device and escape the reservoir 2 through vent 34.

This unexpectedly effective design according to the invention which allows extended use of the reservoir and avoids pressure buildups, is evidenced by the following data which describe a rigorous test in which pressure in the device, as measured at the inlet, remains unusually constant even over extended periods of time.

The precise reasons for the unexpected results achieved with the device of this invention and its mode of operation are not completely understood at the present time. The mode of operation and results described herein are based upon the present understanding of the invention.

Six (6) liters of fresh heparinized bovine blood, adjusted to a 35% hematocrit and an ACT of 420 seconds (±20 sec), was recirculated at 1 LPM for three (3) hours through each unit. Room air was pumped through the unit at 1 LPM along with the blood. The temperature of the blood was ambient room temperature (23°C±2°C). The inlet pressure was monitored at the priming port site (through the defoamer).

Usually, the reservoir of the invention does not show a significant increase in pressure with relation to time, as shown in Table 1.

TABLE 1

| Time (hours) | Average inlet pressure (mmHg) |
|---|---|
| 1 | 2.0 (0.0—3.0) |
| 2 | 3.0 (0.0—4.0) |
| 3 | 3.0 (0.0—4.0) |
| N=4* | |

*N=number of tests

**Claims**

1. A combined cardiotomy defoamer, filter and reservoir device (1, 2) for removing air bubbles and undesirable particulate matter from blood evacuated from a surgical field, said device accommodating substantial blood flow for extended periods of time with minimal pressure increase in said device between the blood inlet (7) and outlet (8) of said device, comprising a hollow, tubular defoamer/filter element (9), a generally hollow housing (3, 4, 5) surrounding said defoamer/filter element (9), and having a blood inlet (7) in communication with the interior of said defoamer/filter element (9), a reservoir chamber (6) for receiving the filtered blood after it has been filtered through said defoamer/filter element (9), an air vent (34) for venting air separated from the blood in said device, and a blood outlet (8) in communication with said reservoir chamber (6), said defoamer/filter element (9) comprising a first annular tube (40) of reticulated porous foam material treated with antifoam compound for removing gas bubbles from said blood and an annular tube (43) of a fine pore size filter screen medium for removing undesirable particulate matter surrounding said first annular tube (40) characterized in that said defoamer/filter element (9) further comprises a second annular tube (41) of a nonwoven fibrous depth filter medium surrounding and closely proximate said first annular tube (40) for providing a first stage filter for larger particulate matter, and a third annular tube (42) of porous material surrounding said second annular tube (41) and positioned between and closely proximate said second annular tube (41) and said annular tube (43) of fine pore size filter screen medium for providing a porous spacing between the external surface of said second annular tube (41) and said downstream fourth annular tube (43) of a fine pore size filter screen medium to provide multiple fluid paths between the exterior surface of said first stage filter (41) and said second stage filter (43).

2. The combined cardiotomy defoamer, filter and reservoir device of claim 1, further characterized in that said second annular tube (41) provides a wide range of non-uniform pore sizes.

3. The combined cardiotomy defoamer, filter and reservoir device of claim 2, further characterized in that said second annular tube (41) has a maximum pore size of approximately 90 microns and a mean pore size of approximately 50 microns.

4. The combined cardiotomy defoamer, filter and reservoir device of claim 1, further characterized in that said fourth annular tube (43) provides a screen filter of well defined, substantially uniform pore size, with said pore size of said fourth annular tube (43) being selected from the range of about 20 to 60 microns.

5. The combined cardiotomy defoamer, filter and reservoir device of claim 1, further characterized in that said second annular tube (41) has a fuzzy inner surface proximate the exterior surface of said first annular tube (40) for providing a larger surface area for blood exiting the porous foam material of tube (40) and for initially capturing the larger particulate matter in said fuzzy material.

6. The combined cardiotomy defoamer, filter and reservoir device of claim 1, further characterized in that said second annular tube (41) is treated with a wetting agent.

7. The combined cardiotomy defoamer, filter and reservoir device of claim 1, further characterized in that said third annular tube (42) is formed of reticulated porous foam material untreated with any antifoam material.

8. The combined cardiotomy defoamer, filter and reservoir device of claim 7, further characterized in that said first annular tube (40) is formed of material having approximately 20 pores per inch and said third annular tube (42) is formed of material having approximately 10 pores per inch.

**Patentansprüche**

1. Kombinierte Kardiotomie-Entschäumer-, Filter- und Speichervorrichtung (1, 2) zur Entfernung von Luftblasen und unerwünschter partikelförmiger Stoffe aus Blut, das aus einem chirurgischen Bereich abgesaugt ist, wobei die genannte Vorrichtung einen beträchtlichen Blutstrom für verlängerte Zeitabschnitte mit minimalem Druckanstieg in der genannten Vorrichtung zwischen dem Bluteinlaß (7) und -auslaß (8) der genannten Vorrichtung, aufnimmt und ein hohles, röhrenförmiges Entschäumer-/Filterelement (9), ein im allgemeinen hohles Gehäuse (3, 4, 5), welches das genannte Entschäumer-/Filterelement (9) umgibt, und einen Bluteinlaß (7) aufweist, der mit dem Inneren des genannten Entschäumer-/Filterelements (9) in Verbindung steht, eine Speicherkammer (6) zur Aufnahme des gefilterten Bluts, nachdem es durch das genannte Entschäumer-/Filterelement (9) gefiltert wurde, eine Entlüftungsöffnung (34) zum Entlüften der von dem Blut in der genannten Vorrichtung getrennten Luft, und einen Blutauslaß (8) in Verbindung mit der genannten Speicherkammer (6), umfaßt, und das genannte Entschäumer-/Filterelement (9) ein erstes ringförmiges Rohr (40) aus vernetztem, porösem Schaummaterial, das mit einer Antischaum-Zusammensetzung behandelt ist, um Gasblasen aus dem genannten Blut zu entfernen und ein ringförmiges Rohr (43) aus einem feinporigen Filtersiebmedium zur Entfernung unerwünschter partikelförmiger Stoffe, welches das genannte erste ringförmige Rohr (40) umgibt, besitzt, dadurch gekennzeichnet, daß das genannte Entschäumer-/Filterelement (9) weiter ein zweites ringförmiges Rohr (41) aus einem nicht-gewebten, faserigen, Tiefenfiltermedium, das das genannte erste ringförmige Rohr (40) umgibt und eng anliegt, um eine erste Filterstufe für größere partikelförmige Stoffe vorzusehen, und ein drittes ringförmiges Rohr (42) aus porösem Material umfaßt, welches das genannte zweite ringförmige Rohr (41) umgibt und zwischen dem genannten zweiten ringförmigen Rohr (41), an diesem eng anliegend, und dem genannten ringförmigen Rohr (43) aus einem feinporigen Filtersiebmedium positioniert ist, um einen porösen Zwischenraum zwischen der äußeren Oberfläche des genannten zweiten ringförmigen Rohres (41) und dem genannten stromabwärtigen vierten ringförmigen Rohr (43) aus einem feinporigen Filtersiebmedium zu bilden, um mehrere Fluidwege zwischen der äußeren Oberfläche des genannten ersten Filters (41) und des genannten zweiten Filters (43) vorzusehen.

2. Kombinierte Kardiotomie-Entschäumer-, Filter- und Speichervorrichtung nach Anspruch 1, weiter dadurch gekennzeichnet, daß das genannte zweite ringförmige Rohr (41) einen weiten Bereich ungleichförmiger Porengrößen vorsieht.

3. Kombinierte Kardiotomie-Entschäumer-, Filter- und Speichervorrichtung nach Anspruch 2, weiter dadurch gekennzeichnet, daß das genannte zweite ringförmige Rohr (41) eine maximale Porengröße von ungefähr 90 µ und eine mittlere Porengröße von ungefähr 50 µ aufweist.

4. Kombinierte Kardiotomie-Entschäumer-, Filter- und Speichervorrichtung nach Anspruch 1, weiter dadurch gekennzeichnet, daß das genannte vierte ringförmige Rohr (43) einen Siebfilter wohl definierter, im wesentlichen gleichförmiger Porengröße liefert, mit einer Porengröße des genannten vierten ringförmigen Rohres (43), die aus dem Bereich von etwa 20 bis 60 µ ausgewählt ist.

5. Kombinierte Kardiotomie-Entschäumer-, Filter- und Speichervorrichtung nach Anspruch 1, weiter dadurch gekennzeichnet, daß das genannte zweite ringförmige Rohr (41) eine flokkige innere Oberfläche nächst der äußeren Oberfläche des genannten ersten ringförmigen Rohres (40) aufweist, um eine größere Oberfläche für das Blut, welches das poröse Schaummaterial des Rohres (40) verläßt vorzusehen, und um anfangs die größeren partikelförmigen Stoffe in dem genannten flockigen Material zu fangen.

6. Kombinierte Kardiotomie-Entschäumer-, Filter- und Speichervorrichtung nach Anspruch 1, weiter dadurch gekennzeichnet, daß das zweite ringförmige Rohr (41) mit einem Benetzungsmittel behandelt ist.

7. Kombinierte Kardiotomie-Entschäumer-, Filter- und Speichervorrichtung nach Anspruch 1, weiter dadurch gekennzeichnet, daß das genannte dritte ringförmige Rohr (42) aus vernetztem, porösem Schaummaterial gebildet ist, das nicht mit irgendeinem Antischaummaterial behandelt ist.

8. Kombinierte Kardiotomie-Entschäumer-, Filter- und Speichervorrichtung nach Anspruch 7, weiter dadurch gekennzeichnet, daß das genannte erste ringförmige Rohr (40) aus Material gebildet ist, welches ungefähr 20 Poren pro Inch aufweist und das genannte dritte ringförmige Rohr (42) aus Material gebildet ist, welches ungefähr 10 Poren pro Inch aufweist.

**Revendications**

1. Dispositif combiné de cardiotomie à casse-mousse, filtre et réservoir (1, 2) pour éliminer des bulles d'air et des particules de matières indésirables du sang évacué d'un champ chirurgical, ledit dispositif convenant à un écoulement important du sang pendant des périodes de temps prolongées avec une élévation minimale de la pression dans ledit dispositif entre l'entrée (7) et la sortie (8) de sang dudit dispositif, comprenant un élément tubulaire creux casse-mousse/filtre (9), un logement globalement creux (3, 4, 5) entourant ledit élément casse-mousse/filtre (9) et ayant une entrée (7) de sang en communication avec l'intérieur dudit élément casse mousse/filtre (9), une chambre (6) de réservoir destinée à recevoir le sang filtré après qu'il a été filtré à travers ledit élément casse-mousse/filtre (9), un évent (34) à air destiné à évacuer l'air séparé du sang dans ledit dispositif et une sortie (8) de sang en communication avec ladite chambre (6) de réservoir, ledit élément casse-mousse/filtre (9) comprenant un premier tube annulaire (40) en mousse poreuse réticulée traitée avec un composé anti-mousse pour éliminer les bulles de gaz dudit sang et un tube annulaire (43) en tamis filtrant à pores de faible dimension pour l'élimination des particules de matières indésirables, entourant ledit premier tube annulaire (40), caractérisé en ce que ledit élément casse-mousse/filtre (9) comprend en outre un deuxième tube annulaire (41) en matière filtrante en profondeur, fibreuse et non tissée, entourant étroitement ledit premier tube annulaire (40) pour constituer un premier étage filtrant destiné aux grosses particules de matières, et un troisième tube annulaire (42) en matière poreuse entourant ledit deuxième tube annulaire (41) et positionné étroitement entre ledit deuxième tube annulaire (41) et ledit tube annulaire (43) en tamis filtrant à pores de faible dimension, pour établir un espace poreux entre la surface extérieure dudit deuxième tube annulaire (41) et ledit quatrième tube annulaire (43) en aval, constitué d'un tamis filtrant à pores de faible dimension, de façon à établir de multiples trajets de fluids entre la surface extérieure dudit premier étage filtrant (41) et ledit second étage filtrant (43).

2. Dispositif combiné de cardiotomie à casse-mousse, filtre et réservoir selon la revendication 1, caractérisé en outre en ce que ledit deuxième tube annulaire (41) présente une large gamme de dimensions non uniforme de pores.

3. Dispositif combiné de cardiotomie à casse-mousse, filtre et réservoir selon la revendication 2, caractérisé en outre en ce que ledit deuxième tube annulaire (41) présente une dimension optimale des pores d'environ 90 micromètres et une dimension moyenne des pores d'environ 50 micromètres.

4. Dispositif combiné de cardiotomie à casse-mousse, filtre et réservoir selon la revendication 1, caractérisé en outre en ce que ledit quatrième tube annulaire (43) constitue un filtre en tamis à pores de dimension bien définie sensiblement uniforme, ladite dimension des pores dudit quatrième tube annulaire (43) étant choisie dans la gamme d'environ 20 à 60 micromètres.

5. Dispositif combiné de cardiotomie à casse-mousse, filtre et réservoir selon la revendication 1, caractérisé en outre en ce que ledit deuxième tube annulaire (41) présente une surface intérieure floconneuse proche de la surface extérieure dudit premier tube annulaire (40) pour établir une plus grande surface spécifique pour le sang sortant de la mousse poreuse du tube (40) et pour retenir initialement les plus grosses particules de matières dans ladite matière floconneuse.

6. Dispositif combiné de cardiotomie à casse-mousse, filtre et réservoir selon la revendication 1, caractérisé en outre en ce que ledit deuxième tube annulaire (41) est traité avec un agent mouillant.

7. Dispositif combiné de cardiotomie à casse-mousse, filtre et réservoir selon la revendication 1, caractérisé en outre en ce que ledit troisième tube annulaire (42) est formé d'une mousse poreuse réticulée non traitée avec une matière anti-mousse quelconque.

8. Dispositif combiné de cardiotomie à casse-mousse, filtre et réservoir selon la revendication 7, caractérisé en outre en ce que ledit premier tube annulaire (40) est formé d'une matière ayant environ 20 pores par inch et ledit troisième tube annulaire (42) est formé d'une matière ayant environ 10 pores par inch.

0 122 748

FIG. 1

FIG. 2

1

FIG. 3

FIG. 4